# EUROPÄISCHE PATENTSCHRIFT

(11) **EP 0 763 520 B1**
(45) Veröffentlichungstag und Bekanntmachung des Hinweises auf die Patenterteilung: **22.12.1999**
(21) Anmeldenummer: 96112940.0
(22) Anmeldetag: 12.08.1996
(51) Int. Cl.: C07C 209/62, C07C 211/47

(54) **Verfahren zur Herstellung von Toluidinen**
Process for the preparation of toluidines
Procédé pour la préparation de toluidines

(30) Priorität: 24.08.1995 DE 19531149
(43) Veröffentlichungstag der Anmeldung: 19.03.1997
(73) Patentinhaber: BAYER AG, 51368 Leverkusen (DE)
(72) Erfinder: Penninger, Stefan, Dr., 50259 Pulheim (DE); Heitkämper, Peter, Dr., 41542 Dormagen (DE)

(56) Entgegenhaltungen:
- US-A- 3 522 310
- US-A- 3 532 754
- US-A- 4 329 501

## Beschreibung

Die vorliegende Erfindung betrifft ein verbessertes Verfahren zur Herstellung von Toluidinen durch reduktive Desaminierung von 2,3- und 3,4-Diaminotoluol.

2,3- und 3,4-Diaminotoluol sind Bestandteile von großtechnisch erzeugtem Toluylendiamin. Bei der üblichen Weiterverarbeitung von Toluylendiamin zu Toluylendiisocyanat würden 2,3- und 3,4-Diaminotoluol, die im folgenden unter der Bezeichnung o-Diaminotoluol zusammengefaßt werden, stören und werden deshalb vorher abgetrennt, im allgemeinen durch Destillation. Dieses abgetrennte o-Diaminotoluol wird zur Herstellung diverser chemischer Produkte verwendet. Jedoch ist die anfallende Menge an o-Diaminotoluol häufig größer, als sie für diese Anwendungen benötigt wird. Es besteht daher die Aufgabe, überschüssiges o-Diaminotoluol wirtschaftlich und ökologisch sinnvoll zu nutzen.

Es ist bekannt, daß sich o-Diaminotoluol katalytisch hydrieren läßt unter Bildung von Toluidinen. Diese Hydrierung verläuft unter Abspaltung und Freisetzung von Ammoniak und wird deshalb als reduktive Desaminierung bezeichnet. Ammoniak und vor allem Toluidine sind wertvolle chemische Grundstoffe, so daß die reduktive Desaminierung von o-Diaminotoluol eine äußerst sinnvolle Umsetzung im Sinne der genannten Aufgabe darstellt.

In der US-Patentschrift 3 532 754 ist ein Verfahren zur Herstellung von o-und m-Toluidin durch reduktive Desaminierung von o-Diaminotoluol beschrieben, das dadurch gekennzeichnet ist, daß man o-Diaminotoluol in Gegenwart von Kobaltoxid-haltigen Trägerkatalysatoren mit Wasserstoff unter erhöhtem Druck bei Temperaturen von 200 bis 250°C umsetzt. Dieses Verfahren ist jedoch mit schweren Nachteilen behaftet.

So liefert dieses Verfahren nur unzureichende Ausbeuten an Toluidin. Wie aus den Beispielen 1 und 4 der genannten Patentschrift hervorgeht, beträgt die Gesamtausbeute an Toluidin bei einer Reaktionstemperatur von 225°C nach 4,5 bzw. 7 Stunden Reaktionszeit nur 52 bzw. 46 % der Theorie, bezogen auf eingesetztes o-Diaminotoluol. Das hat seinen Grund zum Teil darin, daß der Umsatz des eingesetzten o-Diaminotoluols niedrig ist. Beispielsweise beträgt der Umsatz im Beispiel 1 der Patentschrift nach 4,5 Stunden Reaktion bei 225°C nur 64 %.

Im Beispiel 3 der Patentschrift sind für die Umsetzung über 5,25 Stunden bei 225°C Ergebniszahlen genannt, die darauf schließen lassen, daß der Umsatz des eingesetzten o-Diaminotoluols weniger als 20 % beträgt. Da aber eine Reaktionstemperatur von 225°C genau in dem als bevorzugt genannten Bereich von 215 bis 235°C liegt, müssen die in der Patentschrift genannten Kobalt-Katalysatoren für diese Reaktion als ungeeignet angesehen werden, weil sie nur zu unzureichenden Umsätzen des eingesetzten o-Diaminotoluols führen.

Andererseits lehrt die genannte Patentschrift (Spalte 2, Zeilen 23 bis 29), daß die reduktive Desaminiereung von o-Diaminotoluol in Gegenwart der genannten Kobalt-Katalysatoren bei Temperaturen von etwa 250°C zu einer Bildung von Toluol, also zu einer reduktiven Desaminierung und damit zu einer Zersetzung der angestrebten Reaktionsprodukte führt und daß bei noch höheren Reaktionstemperaturen auch Kernhydrierungen unter Bildung von Methylcyclohexylamin und Methylcyclohexan stattfinden. Diese Aussage wird durch die Beispiele der Patentschrift eindrucksvoll bestätigt.

Da Toluol, Methylcyclohexylamin und Methylcyclohexan vergleichsweise wertlose Produkte sind und aufwendig entsorgt werden müssen, ist die Bildung dieser Nebenprodukte bei der reduktiven Desaminierung möglichst zu unterbinden. Das wird nach Aussage der genannten Patentschrift durch Wahl entsprechend niedriger Reaktionstemperaturen erreicht. Damit müssen aber, wie oben ausgeführt, unwirtschaftlich niedrige Umsätze in Kauf genommen werden.

Ein weiterer Nachteil des genannten Verfahrens besteht darin, daß es kein oder praktisch kein p-Toluidin liefert. Wie das Beispiel 1, sowie die Ansprüche 7 und 8 der genannten Patentschrift lehren, ergibt die verfahrensgemäße Umsetzung von o-Diaminotoluol nur o- und m-Toluidin, während p-Toluidin in den Reaktionsgemischen nur in Spuren nachweisbar ist. Dieser Umstand ist aus statistischer Sicht überraschend und läßt vermuten, daß die genannten Kobalt-Katalysatoren bei der Umsetzung selektiv wirken und so die Bildung von p-Toluidin verhindern. Andererseits ist gerade p-Toluidin eine wertvolle chemische Grundsubstanz, so daß die vermutliche Selektivität der Katalysatoren hier zu einem großen Nachteil führt.

Schließlich ist das genannte Verfahren noch mit dem Nachteil behaftet, daß die Umsetzungen hohe Konzentrationen an Kobalt-Katalysatoren erfordern. Als bevorzugt werden in der Patentschrift 30 bis 60 Gew.-Teile Katalysator, bezogen auf 100 Gew.-Teile an eingesetztem o-Diaminotoluol genannt. Diese hohen Konzentrationen führen zwangsläufig zu einer deutlichen Beeinträchtigung der Raum-Zeit-Ausbeuten. Vor allem bedeuten sie einen erheblichen technischen Aufwand, insbesondere bei der Aufarbeitung der Reaktionsgemische, beispielsweise bei der Abtrennung der Katalysatoren durch Filtrieren oder bei der destillativen Auftrennung der Gemische.

In der US-Patentschrift 4 329 501 ist ein weiteres Verfahren zur Herstellung von Toluidinen durch Umsetzung von 2,3- und 3,4-Diaminotoluol mit Wasserstoff unter erhöhtem Druck beschrieben, das dadurch gekennzeichnet ist, daß man als Katalysator Kobalt-Metall verwendet und die Umsetzungen in Gegenwart von Wasser als Lösungsmittel durchführt.

In dieser Patentschrift sind experimentelle Beispiele beschrieben, die die Wirkungsweise des beanspruchten Verfahrens belegen sollen. Die Ergebnisse dieser Beispiele werden in einer Tabelle zusammengefaßt, wobei jedoch unklar ist, welche der beschriebenen Beispiele verfahrensgemäß und welche als Vergleichsbeispiele anzusehen sind. Vor allem werden die Ergebnisse der beschriebenen Versuche nur in Form von gaschromatographisch ermittelten Relativ-Gehalten (Flächen-%) der durch Filtrieren von Katalysator befreiten Reaktionsgemische mitgeteilt, ohne daß deren Absolutmengen und gegebenenfalls deren Wassergehalt angegeben werden. Da auch keine Angaben über die Mengen an wiedergewonnenem Ausgangsprodukt gemacht werden, gibt diese Tabelle weder über die erzielten Umsätze an eingesetztem o-Diamonotoluol noch über die erhaltenen Ausbeuten an gewünschtem Toluidin Auskunft. Damit ist sie für die Beurteilung des beanspruchten Verfahrens wenig aussagekräftig.

Dagegen geht aus dieser Tabelle hervor, daß die Reaktionszeiten der beschriebenen Umsetzungen etwa 10 bis 20 Stunden betragen und damit vergleichsweise hoch sind. Das kann nicht überraschen, da o-Diaminotoluol in einem Lösungsmittel gelöst eingesetzt wird und entsprechend dem Massenwirkungsgesetz sich langsamer umsetzt als in unverdünnter Form.

Aus der US-Patentschrift 3,522,310 ist eine Methode bekannt, nach der man den Gehalt an o-Toluylendiaminisomeren aus einem Toluylendiaminisomerengemisch reduzieren kann. Gemäß der dort beschriebenen Methode wird das Toluylendiaminisomerengemisch bei erhöhter Temperatur und erhöhtem Wasserstoffdruck in Gegenwart eines Hydrierungskatalysators behandelt, wobei sich das Isomerengemisch zugunsten der m-Isomeren verschiebt, die dann isoliert werden können.

Aufgabe der vorliegenden Erfindung ist es daher, ein Verfahren zur Herstellung von Toluidinen durch reduktive Desaminierung von 2,3- und 3,4-Diaminotoluol zur Verfügung zu stellen, das bei hohen Umsätzen des eingesetzten Diaminotoluols zu minimalen Anteilen an Nebenprodukten führt.

Gegenstand der vorliegenden Erfindung ist deshalb ein Verfahren zur Herstellung von Toluidinen durch Umsetzung von 2,3- und/oder 3,4-Diaminotoluol mit Wasserstoff unter erhöhtem Druck in Gegenwart von Katalysatoren, das dadurch gekennzeichnet ist, daß man als Katalysatoren Eisenoxide verwendet und die Umsetzungen bei Temperaturen im Bereich von 260 bis 350 °C sowie bei einem Wasserstoffdruck Von 200 bis 400 bar durchführt.
In das erfindungsgemäße Verfahren können als Edukte chemisch reines 2,3-Diaminotoluol oder 3,4-Diaminotoluol oder Gemische dieser beiden Verbindungen eingesetzt werden. Es können aber auch technische Gemische von 2,3- und 3,4-Diaminotoluol verwendet werden, wie sie beispielsweise bei der destillativen Abtrennung von o-Diaminotoluol aus technischem Toluylendiamin gewonnen werden.

Die erfindungsgemäßen Umsetzungen werden bevorzugt in Abwesenheit von Lösungsmitteln durchgeführt. Jedoch ist die Mitverwendung von inerten Lösungsmitteln bei dem erfindungsgemäßen Verfahren nicht grundsätzlich ausgeschlossen, wenn die besonderen Umstände es erfordern.

Das erfindungsgemäße Verfahren kann diskontinuierlich oder kontinuierlich durchgeführt werden.

Als Katalysatoren für das erfindungsgemäße Verfahren werden z.B. folgende Eisenoxide genannt: FeO (Wüstit), Fe₂O₃, Fe₃O₄, sowie Oxidhydrate des zwei- und/oder dreiwertigen Eisens, beispielsweise Rost. Die Eisenoxide können sowohl einzeln als auch im Gemisch untereinander eingesetzt werden.

Die Menge der für das erfindungsgemäße Verfahren einzusetzenden Eisenoxid-Katalysatoren beträgt üblicherweise 1 bis 12, bevorzugt 3 bis 9 Gew.-Teile, bezogen auf 100 Gew.-Teile an umzusetzendem Diaminotoluol.

Bei dem erfindungsgemäßen Verfahren werden die Katalysatoren in einer oberflächenreichen Form eingesetzt. Beispielsweise können die Katalysatoren für die Umsetzungen in feinkristalliner oder pulverisierter Form in das umzusetzende o-Diaminotoluol eingemischt werden. Es ist jedoch auch möglich, die feinteiligen Eisenoxide auf einem oberflächenreichen Trägermaterial aufzusintern und dieses mit Eisenoxid behaftete Trägermaterial als Festbettkatalysator zu verwenden. Als Trägermaterialien können verwendet werden:
Z.B. Bimsstein, Kieselgur, Silicagel, Alumosilicate oder Aluminiumoxid.

Die Reaktionstemperaturen für das erfindungsgemäße Verfahren betragen 260 bis 350°C, bevorzugt 280 bis 330°C.

Die erfindungsgemäßen Umsetzungen werden üblicherweise bei einem Wasserstoffdruck von 200 bis 400 bar, bevorzugt 250 bis 350 bar durchgeführt.

Die Reaktionszeiten bei dem erfindungsgemäßen Verfahren hängen stark von der gewählten Reaktionstemperatur und der eingesetzten Katalysatormenge ab. Die für die jeweiligen Bedingungen günstige Reaktionszeit läßt sich leicht durch entsprechende Vorversuche ermitteln. Sie beträgt üblicherweise 0,5 bis 10 Stunden. Zweckmäßig werden die Reaktionszeiten so gewählt, daß bei den Umsetzungen praktisch keine Nebenprodukte entstehen. Gegebenenfalls kann es vorteilhaft sein, durch entsprechend kürzere Reaktionszeiten geringfügig kleinere Umsätze in Kauf zu nehmen, um Nebenreaktionen zu vermeiden, wie auch die Beispiele zeigen.

### Beispiele

Es wurde eine Serie von 7 gleichen erfindungsgemäßen Umsetzungen durchgeführt, in denen lediglich die Reaktionszeit variiert wurde. Die Ergebnisse dieser Umsetzungen sind in Tabelle 1 zusammengefaßt.

### Allgemeine Durchführung der Beispiele 1 bis 7

In einem 0,7 l-Rührautoklav wurde ein Gemisch von 146 g 2,3-Diaminotoluol, 254 g 3,4-Diaminotoluol und 25 g pulverisiertem Fe₂O₃ vorgelegt. Der Autoklav wurde mit Stickstoff gespült. Dann wurde das Gemisch bei 300°C und einem Wasserstoffdruck von 260 bar hydriert. Nach Beendigung der gewählten Reaktionszeit wurde der Autoklav entspannt, wobei entstandenes Ammoniak gasförmig entwich. Das Reaktionsgemisch wurde entnommen und zur Abtrennung des Katalysators über eine Drucknutsche filtriert. Die quantitative Analyse erfolgte gaschromatographisch, wobei zur Eichung authentische Substanzproben der einzelnen Komponenten als innere Standards verwendet wurden.

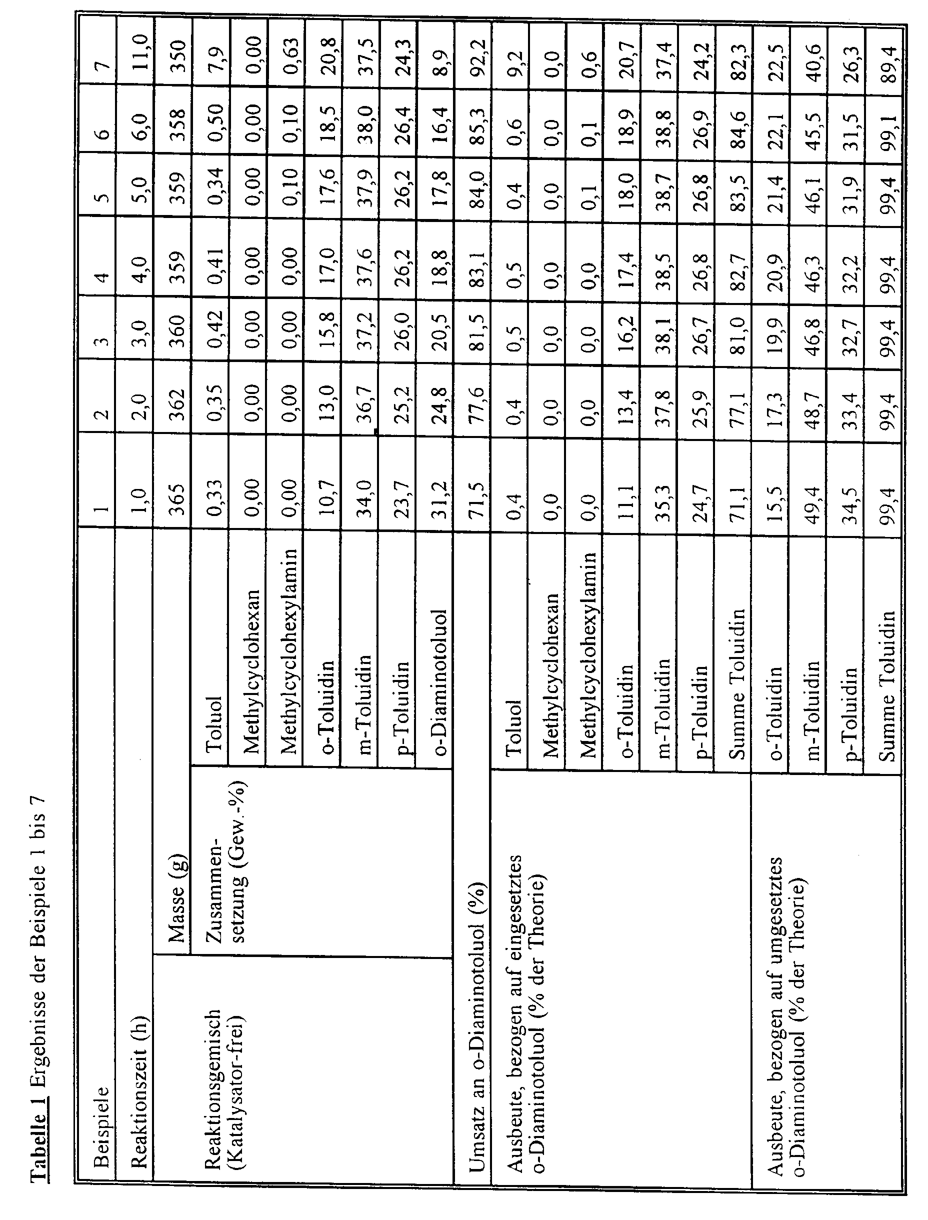

Wie aus Tabelle 1 zu ersehen ist, war es unter den gewählten Bedingungen zweckmäßig, eine Reaktionszeit von weniger als 6 Stunden zu wählen. Zwar konnte bei einer wesentlich größeren Reaktionszeit (Beispiel 7) der Umsatz an o-Diaminotoluol noch merklich gesteigert werden, jedoch im wesentlichen nur zu Gunsten von Nebenprodukten.

## Patentansprüche

1. Verfahren zur Herstellung von Toluidinen durch Umsetzung von 2,3- oder 3,4-Diaminotoluol oder Gemischen von 2,3- und 3,4-Diaminotoluol mit Wasserstoff unter erhöhtem Druck in Gegenwart von Katalysatoren, dadurch gekennzeichnet, daß man als Katalysatoren Eisenoxide verwendet und die Umsetzungen bei Temperaturen im Bereich von 260 bis 350 °C sowie bei einem Wasserstoffdruck von 200 bis 400 bar durchführt.

2. Verfahren nach Anspruch 1, dadurch gekennzeichnet, daß man als Eisenoxide FeO, Fe₂O₃, Fe₃O₄ und/oder Oxidhydrate des 2- und/oder 3-wertigen Eisens einsetzt.

3. Verfahren nach Anspruch 1, dadurch gekennzeichnet, daß man die Eisenoxid-Katalysatoren in einer Menge von 1 bis 12 Gew.-Teilen, bezogen auf 100 Gew.-Teile an umzusetzendem Diaminotoluol, einsetzt.

## Claims

1. Process for the preparation of toluidines by the reaction of 2,3- or 3,4-diaminotoluene or mixtures of 2,3- and 3,4-diaminotoluene with hydrogen under elevated pressure, in the presence of catalysts, characterized in that iron oxides are used as catalysts and the reactions are carried out at temperatures in the range 260 to 350°C and at a hydrogen pressure of 200 to 400 bar.

2. Process according to claim 1, characterized in that the iron oxides used are FeO, Fe₂O₃, Fe₃O₄ and/or hydrated oxides of divalent and/or trivalent iron.

3. Process according to claim 1, characterized in that the iron oxide catalysts are used in an amount of 1 to 12 parts by weight, based on 100 parts by weight of diaminotoluene to be reacted.

## Revendications

1. Procédé pour la préparation de toluidines par réaction du 2,3 ou du 3,4-diaminotoluène ou de mélanges de 2,3 et de 3,4-diaminotoluènes avec l'hydrogène sous pression et en présence de catalyseurs, caractérisé en ce que l'on utilise en tant que catalyseurs des oxydes de fer et en ce que l'on effectue les réactions à des températures dans l'intervalle de 260 à 350°C et des pressions d'hydrogène de 200 à 400 bar.

2. Procédé selon la revendication 1 caractérisé en ce que les oxydes de fer mis en oeuvre sont FeO, Fe₂O₃, Fe₃O₄ et/ou des oxydes hydratés du fer divalent et/ou trivalent.

3. Procédé selon la revendication 1, caractérisé en ce que l'on met en oeuvre les oxydes de fer catalyseurs en quantité de 1 à 12 parties en poids pour 100 parties en poids du diaminotoluène à convertir.
